# EUROPEAN PATENT SPECIFICATION

(11) **EP 3 067 039 B1**
(45) Date of publication and mention of the grant of the patent: **01.07.2020**
(21) Application number: 15819246.8
(22) Date of filing: 15.05.2015
(51) Int. Cl.: A61K 8/58, A61Q 1/12, A61K 8/02

(54) **METHOD FOR PREPARING PRE-MIXED FOUNDATION MATERIAL WITH NEGLIGIBLE CHROMATIC ABBERATION**
HERSTELLUNGSVERFAHREN FÜR EINE FOUNDATIONVORMISCHUNG MIT VERNACHLÄSSIGBAREM CHROMATISCHE ABERRATION
PROCÉDÉ DE PRÉPARATION D'UN PRÉMÉLANGE DE FOND DE TEINT PRÉSENTANT UNE ABBERATION CHROMATIQUE NÉGLIGEABLE

(30) Priority: 10.07.2014 CN 201410326438
(43) Date of publication of application: 14.09.2016
(73) Proprietor: Shanghai Sciencoo Biotech Co., Ltd., Shanghai 200435 (CN)
(72) Inventor: PU, Ke, Shanghai 200435 (CN)
(74) Representative: Ziebig & Hengelhaupt Patent- und Rechtsanwaltskanzlei PartG mbB
(86) International application number: PCT/CN2015/079028
(87) International publication number: WO 2016/004793

(56) References cited:
- CN-A- 101 151 015
- CN-A- 102 481 239
- US-A1- 2011 256 194
- US-A1- 2013 259 914
- Anonymous: "Non-contact spectrophotometer with gloss sensor", , 1 April 2012 (2012-04-01), XP055284053, Retrieved from the Internet: URL:https://www.xrite.com/documents/litera ture/en/L10-407_VS450_4pager_en.pdf [retrieved on 2016-06-28]
- POUX M ET AL: "POWDER MIXING: SOME PRACTICAL RULES APPLIED TO AGITATED SYSTEMS", POWDER TECHNOLOGY, ELSEVIER SEQUOIA, LAUSANNE, CH, vol. 68, 1 January 1991 (1991-01-01), pages 213-234, XP003035641, ISSN: 0032-5910, DOI: 10.1016/0032-5910(91)80047-M

## Description

### Field of the Invention

The present invention relates to a preparation method of a cosmetic material, and more particularly to a method for preparing a pre-mixed foundation material with a negligible chromatic aberration.

### Background of the Invention

Foundation cream/liquid is a very important category of cosmetics, which is also a basis for good makeup effects. By adjusting content of powder with different colors, cosmetic manufacturers usually provide foundation with different colors (such as dark skin color and light skin color) based on user skin colors.

Powder commonly used in foundation cream/liquid comprises titanium dioxide, iron oxide (iron oxide yellow, iron oxide red, iron oxide black, iron oxide brown, etc.), talc, and zinc oxide; in order to improve property indicators such as brightness, feel, and adhesion, functional powder such as boron nitride, bismuth oxychloride, and spherical silica is often added.

In order to fit different formulation systems, or to achieve different functional demands, the powder of the foundation cream/liquid is usually processed with different surface treatments: alkyl silicon treatments, silicon treatments, amino acid treatments, tallate treatments, and so on.

Because of varied types of powder, in order to produce a uniform and stable foundation cream/liquid, the powder to be used in foundation products needs to be pre-mixed and dispersed.

Conventionally, powder pre-mixing and preparation techniques adopted by cosmetic manufacturers are as follows.
1) Mixing with a dispersion medium, and milling through a colloid mill or a three-roll grinding machine;
   wherein the powder to be used in the foundation is pre-mixed with a liquid component (such as glycerin, white mineral oil) listed in the formulations, and then is milled through the colloid mill or the three-roll grinding machine, so as to thoroughly disperse the powder.
2) Pre-mixing with a high-speed mixer;
   wherein the powder to be used in the foundation is pre-mixed by the high-speed mixer, which is conducive to subsequent use.
3) Purchasing color powder (such as iron oxide) of a same batch in a large quantity, so as to ensure small chromatic aberration within a certain period of time.

All the above three preparation techniques have great defects in practice.
1) Defects of Mixing with a dispersion medium, and milling through a colloid mill or a three-roll grinding machine:
   A) the powder is forcibly dispersed by a physical method, so surface property difference of the powder is not changed, which is easy to cause secondary aggregation, or color separation;
   B) only a dispersion state of the powder is improved, which is not conducive to control the chromatic aberration; instead, because of more processes, the chromatic aberration is more difficult to control;
   C) it is easy to introduce secondary pollution, particularly metal pollution; wherein portions, which contact with materials, of a high-quality three-roll grinding machine or colloid mill must be made of high quality stainless steel, and surfaces thereof must be specially treated; otherwise, surfaces of titanium dioxide (which is a main component having a best covering effect in foundation) will often be wrapped by inorganic materials such as alumina and zirconia; wherein such inorganic materials have high hardness (for example, aluminum oxide, commonly known as corundum, has a hardness second only to diamond), and are able to easily wear the stainless steel; and iron dust caused by wear will bring pollution to the foundation;
      furthermore, the high-quality colloid mill or three-roll grinding machine is expensive, and most small and medium cosmetic companies just use common-quality grinding equipments, which is not able to prevent secondary pollution.
2) defects of pre-mixing with a high-speed mixer:
   A. usually, the high-speed mixer only physically mixing the materials with a limited efficiency;
      because common high-speed mixer is limited by size and motor power, a stirring speed is impossible to be very high (wherein for a 50-100L mixer, a maximum rotation speed is only 1500rpm), so it is difficult to completely dispersing the powder;
      during stirring, some companies will spray components such as silicone oil and silane coupling agent, so as to process the powder with simpler surface treatments, which homogenizes the surface properties of varied powder to some extent, and improves the dispersion of the powder; however, due to a poor uniformity caused by such simple dry treatment, an application scope is narrow (because a lot of surface treatments are not suitable for dry treatments), resulting in a limited improvement;
   B) there is no improvement for chromatic aberration control.
3) Defects of purchasing color powder of a same batch in a large quantity:
   A) a lot of money will be occupied and a lot of inventories will be backlogged; furthermore, the powder of same batch will run out, which is not able to fundamentally solve the problem, and is also not suitable for small and medium cosmetic companies;
   B. chromatic aberration of the foundation is related not only with the batch, but also with a variety of factors such as the surface properties and the dispersion state of the powder; therefore, purchasing color powder of the same batch in a large quantity only improves the chromatic aberration to some extent, and is not able to solve the problem fundamentally.

Foundation cream/liquid forms an important makeup category, so color stability thereof is critical. However, the powder used is varied, which has great physical and chemical property differences such as size, shape, density and surface property; wherein proportions of the varied powder, surface properties, dispersion and distribution states in the formulation will all affect color performances of the foundation. Moreover, there is chromatic aberration between different batches of powder products of titanium dioxide and iron oxide. Therefore, a chromatic aberration problem of the foundation products is so difficult to be solved that a lot of people think the problem is unsolvable.

US 2013/259914 A1 discloses lauric lysine treatment of a platy substrate, wherein the plated substrate is one or more of synthetic mica; metal oxide-coated synthetic mica; platy aluminum oxide flakes; metal oxide-coated platy aluminum oxide flakes; silicon dioxide flakes; metal oxide-coated silicon dioxide flakes; glass flakes; metal oxide-coated glass flakes; perlite flakes; and metal oxide-coated perlite flakes. The treated play substrates may be used as pigments for cosmetics.

### Summary of the Invention

A technical problem to be solved by the present invention is to provide a method for preparing a pre-mixed foundation material with a negligible chromatic aberration. The present invention fundamentally overcomes defects of the above technologies, so as to enable manufacturers to easily produce foundation products with negligible chromatic aberration (ΔE_{cmc} < 0.8, which is unable to be distinguished by naked eyes).

Accordingly, in order to accomplish the above objects, the present invention provides:
a method for preparing a pre-mixed foundation material with a negligible chromatic aberration , the method comprising the steps of:
(1) processing all raw material powders of the pre-mixed foundation material with uniform surface treatments, so as to homogenize surface properties of the raw material powders, the surface treatments comprise alkyl silicon treatments or lauric lysine treatments, and the inorganic pigment comprises iron oxide yellow, iron oxide red and iron oxide black;
(2) after the surface treatments, thoroughly stirring the raw material powder with a high speed for obtaining the pre-mixed foundation material; and after mixing with a high-speed mixer, post-treating the pre-mixed foundation material by a jet milling method, so as to evenly disperse powder of the pre-mixed foundation material;
(3) determining of a chromatic aberration ΔE_{cmc} of the pre-mixed foundation material compared to a standard sample, wherein a non-contact chromatic aberration meter is used for precisely measuring the chromatic aberration of the pre-mixed foundation material; and
(4) if the chromatic aberration ΔE_{cmc} is not < 0.8, adjusting a color with a pre-prepared color concentrate, wherein the pre-prepared color concentrate is pre-prepared with the step (1) and the step (2).

According to the method, in the step (1), the surface treatments homogenizes the surface properties of the raw material powder, so as to easily achieve even dispersion, wherein conventional surface treatments are all adopted;, the surface treatments comprises alkyl silicon treatments or lauric lysine treatments.

A method of the alkyl silicon treatments comprises steps of:
(1) thoroughly mixing 0.45kg octyl triethxy silane with 1.05kg 95% ethanol for obtaining a surface treatment agent just before using;
(2) adding 15kg the raw material powder into the high-speed mixer; under a high mixing speed, spraying the surface treatment agent prepared into the high-speed mixer with a spray tank, and thoroughly stirring for thoroughly mixing the surface treatment agent with the raw material powder; and
(3) discharging the raw material powder and instantly sending into an oven, heating at 70-105°C for 4h, and naturally cooling before discharging.

A method of the lauric lysine treatments comprises steps of:
(1) pre-dispersing the raw material powder into a 10%-15% water solution;
(2) adjusting a pH value to about 12 by sodium hydroxide;
(3) adding 5-10wt% lauric lysine powder;
(4) increasing a solution temperature to 80°C and stirring for 30min for mixing;
(5) adjusting the pH value to 6 by sulphuric acid;
(6) keeping the temperature, then stirring and reacting for 30min; and
(7) washing, drying, and crashing.

According to the method, in the step (2), the jet milling method comprises processing the pre-mixed foundation material with 7-8Mpa high-pressure air.

According to the method, in the step (4), the pre-prepared color concentrate comprises a yellow concentrate, a red concentrate and a black concentrate; wherein the yellow concentrate is formed by 60wt% titanium dioxide and 40wt% iron oxide yellow; the red concentrate is formed by 60wt% titanium dioxide and 40wt% iron oxide red; the black concentrate is formed by 80wt% titanium dioxide and 20wt% iron oxide black.

A pre-mixed foundation material prepared by the method has a chromatic aberration ΔE_{cmc} < 0.8. The pre-mixed foundation material is able to be used in products such as foundation cream/liquid in a large quantity, so as to solve the chromatic aberration problem between different batches.

Accordingly, technical effects of the present invention are as follows:
1) with help of instruments, the chromatic aberration is digitally and quantitatively measured;
2) varied powder is processed with the uniform surface treatments, so as to enable the uniform surface properties, resulting in even dispersion in a dispersion medium or an emulsification system;
3) the jet milling method is used, so as to ensure that the powder is thoroughly dispersed without bond aggregation;
4) with the color concentrate which is processed with the surface treatments and the jet milling, the color of the pre-mixed material with the chromatic aberration is finely adjusted; and
5) by whole-process monitor of each pot of the color powder, and by mixing in a large quantity with a spiral ribbon mixer, the colors of the pre-mixed material in different batches are homogenized.

### Brief Description of the Drawings

Fig. 1 illustrates reaction processes of a powder surface treatment.
Fig. 2 illustrates powder surface chemical properties.
Fig. 3 is photos of LL-treated powder.
Fig. 4 is a microscope image of a Si4254 pre-mixed material with 10% white mineral oil dispersion liquid after being crashed by jet milling.
Fig. 5 a microscope image of the Si4254 pre-mixed material with the 10% white mineral oil dispersion liquid before being crashed by the jet milling.
Fig. 6 illustrates a test interface of VS450.
Fig. 7 illustrates a test result of the VS450.
Fig. 8 illustrates comparison between standard colors and tested colors with a profession displayer.
Fig. 9 illustrates a chromatic aberration between tested samples and standard samples.
Fig. 10 illustrates color comparison between dried powder (compressed powder) of the Si4254 pre-mixed material and the standard samples.
Fig. 11 illustrates a chromatic aberration test result of a foundation cream produced with the Si4254 pre-mixed material.

### Embodiments

Referring to preferred embodiment, the present invention is further illustrated.

According to the present invention, by analyzing and cracking varied factors which affect a foundation chromatic aberration, a complete technical method is finally formed, wherein a pre-mixed foundation material with a negligible chromatic aberration is prepared, so as to prepare a foundation product with a negligible chromatic aberration. Specifically, the method comprises steps as follows.
(1) Processing all raw material powder of the pre-mixed foundation material with uniform surface treatments, so as to homogenize surface properties of the raw material powder.
For ensuring sufficient dispersion of varied powder in a dispersion medium or an emulsification system and thereby controlling the chromatic aberration of the foundation production, the powder must be processed with uniform surface treatments, so as to homogenize surface properties of the varied powder with great physical and chemical property differences.

**table 1**

| model | particle shape/molecular formula/purity | average particle size | specific area (m²/g) | color | stacking density (g/cc) | specific weight (g/cc) | pH |
|---|---|---|---|---|---|---|---|
| iron oxide red | | | | | | | |
| R-2199 | spherical/Fe₂O₃/99+ | 0.27 µm | 18.0 | red | 0.90 | 5.15 | 7 |
| | | | | L*=41.66 | | | |
| | | | | a*=35.66 | | | |
| | | | | b*=30.31 | | | |

| iron oxide yellow | | | | | | | |
|---|---|---|---|---|---|---|---|
| YP-1750 | acicular/FeOOH/99 | 0.65 µm | 14.0 | yellow | 0.35 | 4.30 | 6.5 |
| | | | | L*=70.23 | | | |
| | | | | a*=15.07 | | | |
| | | | | b*=58.22 | | | |

| iron oxide black | | | | | | | |
|---|---|---|---|---|---|---|---|
| BK-5000 | cubical/Fe₃O₄/99 | 0.30 µm | 8.0 | black | 0.80 | 5.00 | 8 |
| | | | | L*=36.59 | | | |
| | | | | a*=1.78 | | | |
| | | | | b*=-1.02 | | | |

| titanium dioxide | | | | | | | |
|---|---|---|---|---|---|---|---|
| R902+ | spherical/TiO₂/93 | 0.40 µm | | white | | 4.00 | 7.9 |

Referring to table 1, physical and chemical properties of four essential kinds of powder in a simplest pre-mixed foundation material are listed. It can be concluded that there are huge differences in aspects such as powder shapes, particle sizes, specific areas, and specific weights.
Without the uniform surface treatments, the varied powder in the pre-mixed foundation material is difficult to be evenly distributed in a dispersion medium or a formulation system, not to mention controlling the chromatic aberration.
Two preferred surface treatments techniques are provided as follow:
1.1) a method of a alkyl silicon treatments comprises steps of
   thoroughly mixing 0.45kg octyl triethxy silane with 1.05kg (95%) ethanol for obtaining a surface treatment agent just before using;
   adding 15kg powder (such as titanium dioxide, iron oxide yellow, iron oxide red and iron oxide black) into the high-speed mixer; under a high mixing speed, spraying the surface treatment agent prepared into the high-speed mixer with a spray tank, and thoroughly stirring for thoroughly mixing the surface treatment agent with the powder; and
   discharging the raw material powder and instantly sending into an oven, heating at 105°C (or 70°C for the iron oxide yellow) for 4h, and naturally cooling before discharging.

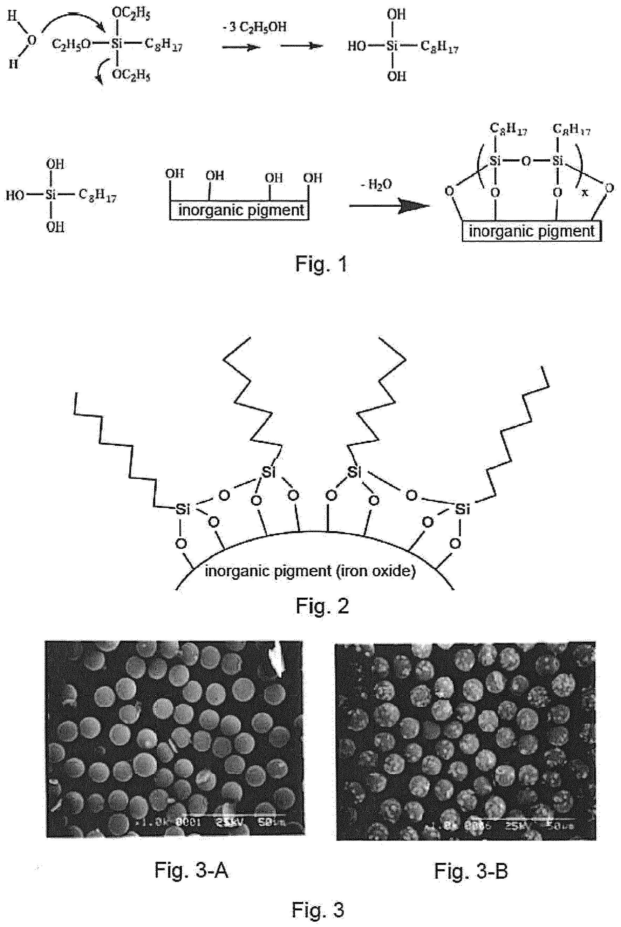

   Fig. 1 illustrates reaction processes of the method; and fig. 2 illustrates powder surface chemical properties after being treated.
   It can be concluded that after the uniform surface treatments, the surface properties of the inorganic pigments and the titanium dioxide are very similar, which is conducive to achieve a homogenized distribution state in the dispersion medium or the formulation system.
1.2) a method of a lauric lysine treatments (LL-treatment for short) comprises steps of:
   (1) pre-dispersing the raw material powder into a 10%-15% water solution;
   (2) adjusting a pH value to about 12 by sodium hydroxide;
   (3) adding 8wt% lauric lysine powder;
   (4) increasing a solution temperature to 80°C and stirring for 30min for mixing;
   (5) adjusting the pH value to 6 by sulphuric acid;
   (6) keeping the temperature, then stirring and reacting for 30min; and
   (7) washing, drying, and crashing.

Fig. 3 is photos of LL-treated powder, wherein 3-A illustrates untreated powder and 3-B illustrates treated powder.
With a certain proportion, the above treated titanium and iron oxide are able to be mixed through a high-speed mixer for forming the pre-mixed foundation material. For example, an oil-dispersed pre-mixed foundation material 1# comprises (wt%):
alkyl silicon treated (or LL-treated) titanium dioxide: 88%,
alkyl silicon treated (or LL-treated) iron oxide yellow: 7.6%,
alkyl silicon treated (or LL-treated) iron oxide red: 3%,
alkyl silicon treated (or LL-treated) iron oxide black: 1.4%.

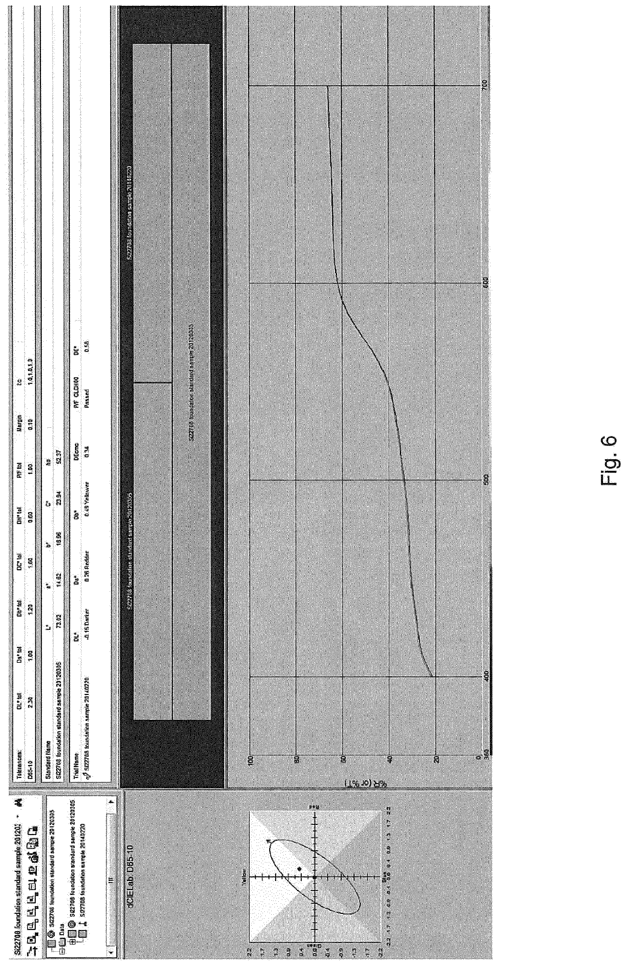

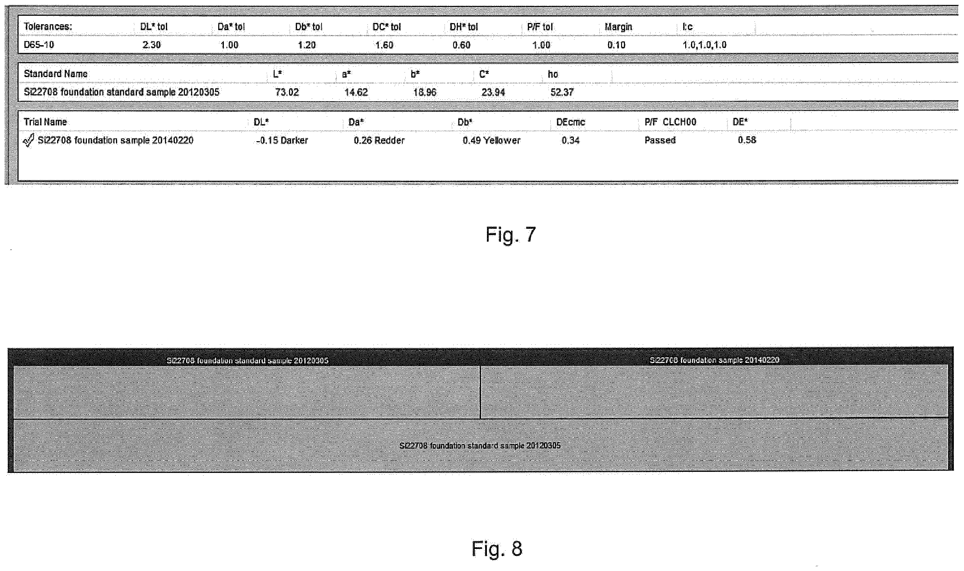

(2) After the surface treatments, thoroughly stirring the raw material powder with a high speed for obtaining the pre-mixed foundation material; and after mixing with a high-speed mixer, post-treating the pre-mixed foundation material by a jet milling method, so as to evenly disperse powder of the pre-mixed foundation material.
For ensuring stable colors and controllable chromatic aberration, all powder, especially the color powder, must be under (or almost under) an ideal dispersion state without aggregated or bonded powder.
If the powder (especially the color powder) is bonded together, a total color of the powder will not be fully demonstrated; in subsequent production process foundation products, there may be grinding, homogenizing and other processes, which may break up the powder which is originally bonded together, resulting in great disturbance of color stability.
In order to ensure sufficient dispersion, the present invention post-treats the pre-mixed material from the high-speed mixer with the jet milling method comprising:
blowing the powder for crashing with each other by filtered and dried high-pressure air, so as to separate the bonded powder. According to the method, a pressure of the high-pressure air is limited (at 7-8Mpa), which generally only separates the bonded powder rather than changes an original particle diameter and size.

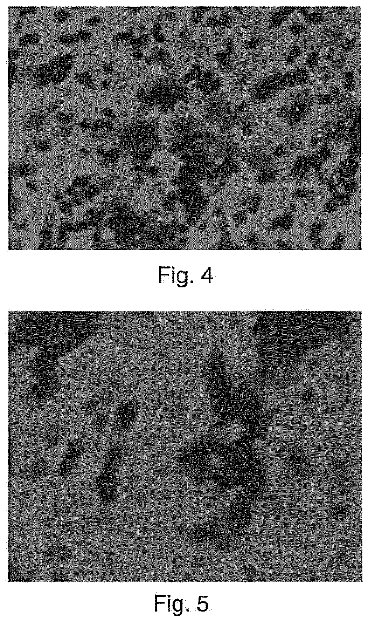

Fig. 4 is a microscope image of a Si4254 pre-mixed material with 10% white mineral oil dispersion liquid after being crashed by jet milling, wherein the powder is evenly dispersed.
Fig. 5 a microscope image of the Si4254 pre-mixed material with the 10% white mineral oil dispersion liquid before being crashed by the jet milling, wherein there is an obvious powder aggregation phenomenon.
(3) Measuring the chromatic aberration, wherein a non-contact chromatic aberration meter is used for precisely measuring the chromatic aberration of the pre-mixed foundation material.
For controlling the chromatic aberration, firstly, a chromatic aberration degree, a shifting direction (to red or yellow), and a shifting value of the color must be determined. Without precisely measuring the chromatic aberration, the chromatic aberration cannot be controlled only by senses of naked eyes.
The pre-mixed foundation material (in a powder form) and the foundation cream/liquid (in a cream or liquid form) have non-transparent special states, whose chromatic aberration is hard to be directly measured by conventional detection instruments.
According to the present invention, a most advanced non-contact chromatic aberration meter VS450 from US Xrite Company is used, which is able to precisely and conveniently measuring the chromatic aberration: wherein fig. 6 illustrates a test interface of the VS450.
Referring to fig. 7, color depths and color shifting directions when being compared with standard samples are clearly presented. According to the present invention, a qualified produced pre-mixed foundation material has a DE_{cmc} (i.e. ΔE_{cmc}) less than 0.8. Referring to fig. 7, the DE_{cmc} is only 0.34, which is much lower than a standard.
Fig. 8 illustrates comparison between standard colors and tested colors with a profession displayer. Another advantage of digitally determining the color is: standard colors are converted into digital files and then stored in a computer, which will not be changed if time or sampler properties have be changed.

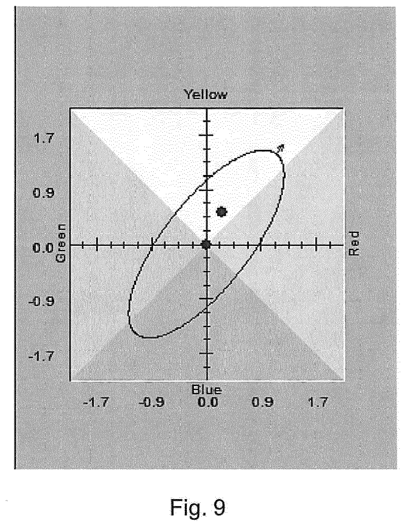

Fig. 9 directly illustrates a chromatic aberration between tested samples and standard samples: wherein a center point of a coordinate axis is the standard sample, a black point at a top-right first quadrant is the tested sample; inner area of a blue oval means qualified. Referring to the fig. 9, the tested sample relatively shifts to yellow and red.
(4) Adjusting a color with a pre-prepared color concentrate, wherein the pre-prepared color concentrate is pre-prepared with the step (1) and the step (2).
With the above method, the chromatic aberration is precisely measured while the sufficient dispersion state of the powder is ensured. However, the chromatic aberration is inevitable.
It must be admitted that the chromatic aberration objectively exists. Even color powder (such as iron oxide) with best quality and highest purity cannot eliminate the chromatic aberration. For ensuring color stability of the pre-mixed material provided by the present invention, color adjustment is necessary.
There is a key point: the color must be adjusted by color concentrates in the ideal dispersion state and cannot be adjusted directly by the color powder, which is essential for ensuring the ideal dispersion state of the color powder to be added into the pre-mixed material. Otherwise, the color adjustment will fail.

**table 2: proportions of color concentrates used (wt%)**

| component name | yellow concentrate | red concentrate | black concentrate |
|---|---|---|---|
| alkyl silicon treated (or LL-treated) titanium dioxide | 60 | 60 | 80 |
| alkyl silicon treated (or LL-treated) iron oxide yellow | 40 | | |
| alkyl silicon treated (or LL-treated) iron oxide red | | 40 | |
| alkyl silicon treated (or LL-treated) iron oxide black | | | 20 |

(5) Monitoring the chromatic aberration during the whole processes, and mixing in a large quantity.

After finely adjusting the color, it can be guaranteed that a small quantity of the pre-mixed materials is identical to the standard samples. However, due to a limited production ability of the high-speed mixer, a yield is 20-30kg/batch. Therefore, color monitor in subsequent production of is necessary.

Fortunately, it has been noticed through long-term production that after a first color adjustment, subsequence products with proportions equaling to that of the first color adjustment have sufficient color stability and repeatability, which is due to the ideal dispersion states of the pre-mixed material and the color concentrate.

In production practice, emphasis is put on first three batches. If the color keeps stable, the subsequent batches will be monitor randomly.

After multi-batch production, a spherical ribbon mixer is used for mixing 200-400kg the pre-mixed material again for obtaining a unified production batch, so as to ensure complete homogeneity of the pre-mixed materials in a same batch.

According to the present invention, by techniques such as digitally and quantitatively analyzing the chromatic aberration, the uniform surface treatments of the varied powder, solving a powder agglomeration problem, and finely adjusting the color powder, the pre-mixed foundation materials are produced. The pre-mix material has the negligible chromatic aberration (i.e. ΔE_{cmc} < 0.8; far less than a chromatic aberration range which is sensible by naked eyes), and is able to be easily used, which helps cosmetic manufacturers to easily produce foundation cream/liquid with the negligible chromatic aberration. Production costs are greatly reduced while production efficiency is improved.

According to the above method, several adoptable proportions of the color-changeable pre-mixed foundation material are provided as follows:

| component name | Si4254 | Si22708 | Si883 | Si04BB |
|---|---|---|---|---|
| alkyl silicon treated (or LL-treated) titanium dioxide | 82.3 | 90.3 | 79.8 | 83.6 |
| alkyl silicon treated (or LL-treated) iron oxide yellow | 10.7 | 4.5 | 11.9 | 10.7 |
| alkyl silicon treated (or LL-treated) iron oxide red | 3.5 | 4.3 | 4.1 | 2.3 |
| alkyl silicon treated (or LL-treated) iron oxide black | 3.5 | 0.9 | 4.2 | 3.4 |

A unit of the above proportions is wt%.

The raw material powder with the above proportions is prepared into the pre-mixed foundation material with the method of the present invention, and the pre-mixed foundation material is prepared into a foundation product. According to the present invention, an embodiment is: color-coagulated lotion foundation cream.
1) Formulation: BB007W/O

| phase number | component name | content (Wt)% | manufacturer/provider |
|---|---|---|---|
| A | Abil EM90 | 2.00 | Degussa |
| | LAMEFORM® TGI | 2.00 | Corning |
| | LIPONATE TDS | 8.00 | Herbalife |
| | LIPONATE TDTM | 2.00 | Herbalife |
| | LIPOVOL GTB | 1.50 | Herbalife |
| | Silsoft 034 | 3.00 | MTO |
| | artificial squalane | 7.00 | Japan NOF |
| | dolomol | 0.50 | |
| B | Si4254 (pre-mixed foundation material) | 19.00 | |
| | bismuth oxychloride | 2.00 | UNI-powder |
| | silicon-treated 1250-mesh mica powder | 3.00 | UNI-powder |
| C | deionized water | to 100 | |
| | glycerol | 5.00 | Coconut Tree |
| | lithium magnesium silicate | 0.60 | Hemings |
| | 1,3 -butanediol | 8.00 | |
| | MgSO4*7H2O | 0.70 | AR |
| D | Glydant Plµs | 0.30 | LONZA |
| | essence | appropriate amount | |

2) Preparation method:
heating an A phase to 75-80°C, then adding a powder material of a B phase, homogenizing (with 10000rpm) for 30min, so as to thoroughly disperse the powder; meanwhile, heating a C phase to 75-80°C for completely melting;
increasing a stirring speed of the A and B phases (to 500-600rpm), slowly adding the C phase into the A and B phases for completely emulsifying; and homogenizing (with 10000rpm) for 1min; and
cooling by stirring (with 380-400rpm) until a temperature is lower than 45°C, then adding a D phase and stirring, then discharging.

Verification of effects of the pre-mixed foundation powder of the present invention:

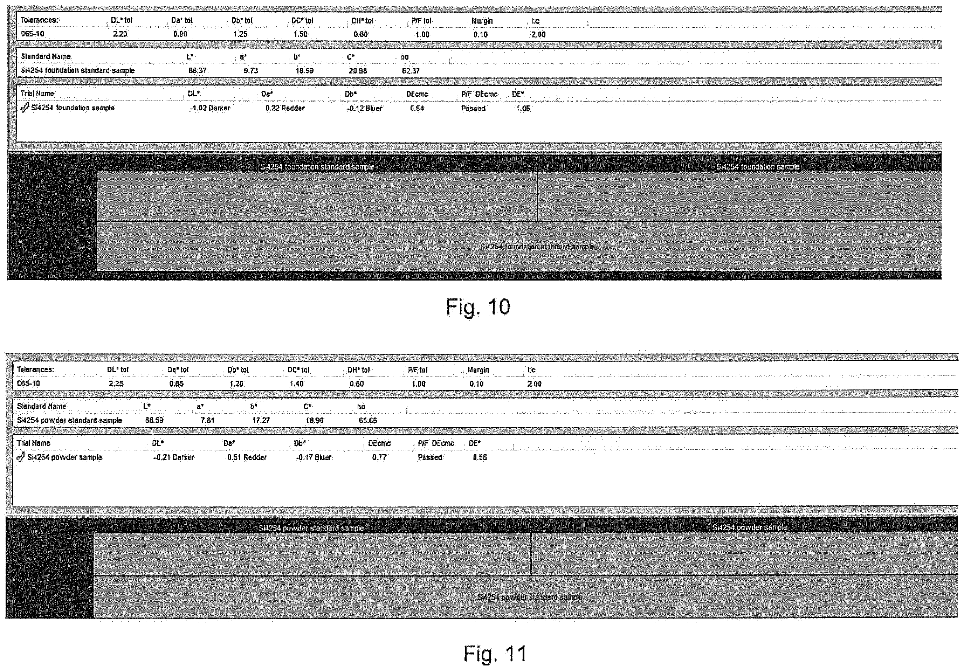

Fig. 10 illustrates color comparison between dried powder (compressed powder) of the Si4254 pre-mixed material and the standard samples.

Accordingly, a color of a Si4254, Mar. 2014 was quite similar (i.e. ΔE_{cmc} < 0.8) to a color of a standard Si4254, Apr. 2012, wherein multi-batch production for 2 years, a color stability stays hige.

Fig. 11 illustrates a test result of a foundation cream produced with the Si4254.

Accordingly, the chromatic aberration of foundations produced with the pre-mixed foundation material in a same tested batch is highly controllable, whose ΔE_{cmc} is about 0.54 and is far less than a standard value of 0.8, which is almot unable to be distinguish by naked eyes, and may be described as a "zero chromatic aberration" foundation.

About ΔE, quality standards of major iron oxide manufacturers in the world are as follows: United States Sun Chemical: ΔE < ±1.4; France Sensient LCW, which is famous for small chromatic aberration and high purity: ΔE < 1.2; and Lockwood United States with a highest purity of iron oxide: ΔE < 1.0. Above are chromatic aberrations of a single color (such as iron oxide yellow). Thus, for the pre-mixed foundation material of the present invention with a variety of colors, it is indeed commendable to reach ΔE_{cmc} < 0.8.
[Note: CMC (Color Measurement Committee) is a British association of dyes and pigments, which made an oval ΔE formula in a C1ELAB color space. With a tolerance method of the CMC, visual scope of a chromatic aberration is an oval, and results of the method are more similar to that of human eyes. Therefore, many industries believe ΔE_{cmc} is more accurate than ΔE for describing the chromatic aberration.]

Based on the above preferred embodiment, advantages of the present invention are as follows:
1) Product colors are stable, and chromatic aberration between difference batches is quite small and is highly consistent with standard samples.
2) Utilization is easy, production is simple, and production efficiency is greatly improved.
   Due to sufficient surface treatments and being crashed by jet milling, the pre-mixed foundation material prepared by the method of the present invention is easily dispersed in the dispersion medium without grinding equipment such as three-roll grinding machine and colloid mill, which greatly improves the production efficiency.
3) Colors are varied, and powder ingredients are flexible and changeable for achieving different make-up effects.

The color of the pre-mixed foundation material prepared by the method of the present invention may be dark or light. Powder such as boron nitride and bismuth oxychloride may be added, which will not affect controllability of the chromatic aberration and convenience of utilization.

### Industrial practicability

The pre-mixed foundation material prepared by the method of the present invention h can reach a rigid chromatic aberration standard, i.e. ΔE_{cmc} < 0.8, and is able to be used in products such as the foundation cream/liquid in a larger quantity, so as to solve the chromatic aberration problem between different batches.

Technical effects of the present invention are achieved: with the help of instruments, quantitative analysis of digital color size; unification of various powder surface treatment, it has a uniform surface properties, resulting in a dispersion medium or distributed uniformly dispersed emulsion system; using jet milling methods to ensure that the powder thoroughly dispersed without bond aggregation; the use of surface-treated and airflow crushed "masterbatch", to have the color of premix color trim; by the color of each pot powder process monitoring, and use ribbon mixer mixing large quantities to ensure that each batch of premix uniform color, which enables a great market prospect and a strong industrial practicability.

## Claims

1. A method for preparing a pre-mixed foundation material with a negligible chromatic aberration, comprising steps of:
(1) processing all raw material powders of the pre-mixed foundation material with uniform surface treatments, so as to homogenize surface properties of the raw material powders, wherein the raw material powders comprise inorganic pigments and titanium dioxide, the surface treatments comprise alkyl silicon treatments or lauric lysine treatments, and the inorganic pigment comprises iron oxide yellow, iron oxide red and iron oxide black;
(2) after the surface treatments, thoroughly stirring the raw material powders with a high speed for obtaining the pre-mixed foundation material; and after mixing with a high-speed mixer, post-treating the pre-mixed foundation material by a jet milling method, so as to evenly disperse powder of the pre-mixed foundation material;
(3) determining of a chromatic aberration ΔE_{cmc} of the pre-mixed foundation material compared to a standard sample, wherein a non-contact chromatic aberration meter is used for precisely measuring the chromatic aberration of the pre-mixed foundation material; and
(4) if the chromatic aberration ΔE_{cmc} is not < 0.8, adjusting a color with a pre-prepared color concentrate, wherein the pre-prepared color concentrate is pre-prepared with the step (1) and the step (2).

2. The method, as recited in claim 1, wherein a method of the alkyl silicon treatments comprises steps of:
(1) thoroughly mixing 0.45kg octyl triethxy silane with 1.05kg 95% ethanol for obtaining a surface treatment agent just before using;
(2) adding 15kg the raw material powder into the high-speed mixer; under a high mixing speed, spraying the surface treatment agent prepared into the high-speed mixer with a spray tank, and thoroughly stirring for thoroughly mixing the surface treatment agent with the raw material powder; and
(3) discharging the raw material powder and instantly sending into an oven, heating at 70-105°C for 4h, and naturally cooling before discharging.

3. The method, as recited in claim 1, wherein a method of the lauric lysine treatments comprises steps of:
(1) pre-dispersing the raw material powder into a 10%-15% water solution;
(2) adjusting a pH value to about 12 by sodium hydroxide;
(3) adding 5-10wt% lauric lysine powder;
(4) increasing a solution temperature to 80°C and stirring for 30min for mixing;
(5) adjusting the pH value to 6 by sulphuric acid;
(6) keeping the temperature, then stirring and reacting for 30min; and
(7) washing, drying, and crashing.

4. The method, as recited in claim 1, wherein in the step (2), the jet milling method comprises processing the pre-mixed foundation material with 7-8Mpa high-pressure air.

5. The method, as recited in claim 1, wherein in the step (4), the pre-prepared color concentrate comprises a yellow concentrate, a red concentrate and a black concentrate; wherein the yellow concentrate is formed by 60wt% titanium dioxide and 40wt% iron oxide yellow; the red concentrate is formed by 60wt% titanium dioxide and 40wt% iron oxide red; the black concentrate is formed by 80wt% titanium dioxide and 20wt% iron oxide black.

## Patentansprüche

1. Verfahren zur Herstellung eines vorgemischten Grundierungsmaterials mit einer vernachlässigbaren Farbabweichung, umfassend die folgenden Schritte:
(1) Bearbeiten aller Rohmaterialpulver des vorgemischten Grundierungsmaterials mit einheitlichen Oberflächenbehandlungen, um die Oberflächeneigenschaften der Rohmaterialpulver zu homogenisieren, wobei die Rohmaterialpulver anorganische Pigmente und Titandioxid umfassen, die Oberflächenbehandlungen Behandlungen mit Alkylsilicium oder Behandlungen mit laurinsäurehaltigem Lysin umfassen, und das anorganische Pigment Eisenoxidgelb, Eisenoxidrot und Eisenoxidschwarz umfasst;
(2) nach den Oberflächenbehandlungen, gründliches Rühren der Rohmaterialpulver mit einer hohen Geschwindigkeit zum Erhalten des vorgemischten Grundierungsmaterials; und nach dem Mischen mit einem Hochgeschwindigkeitsmischer, Nachbehandeln des vorgemischten Grundierungsmaterials mit einem Strahlmahlverfahren, um das Pulver des vorgemischten Grundierungsmaterials gleichmäßig zu dispergieren;
(3) Bestimmen einer Farbabweichung ΔE_{cmc} des vorgemischten Grundierungsmaterials gegenüber einer Standardprobe, wobei ein berührungsloses Farbabweichungsmessgerät zur präzisen Messung der Farbabweichung des vorgemischten Grundierungsmaterials verwendet wird; und
(4) wenn die Farbabweichung ΔE_{cmc} nicht < 0,8 ist, Einstellen einer Farbe mit einem vorher hergestellten Farbkonzentrat, wobei das vorher hergestellte Farbkonzentrat mit dem Schritt (1) und dem Schritt (2) vorher hergestellt wird.

2. Verfahren nach Anspruch 1, wobei ein Verfahren der Behandlungen mit Alkylsilicium die folgenden Schritte umfasst:
(1) gründliches Mischen von 0,45 kg Octyltriethoxysilan mit 1,05 kg 95%igem Ethanol zum Erhalten eines Oberflächenbehandlungsmittels unmittelbar vor der Verwendung;
(2) Hinzugeben von 15 kg des Rohmaterialpulvers in den Hochgeschwindigkeitsmischer; unter einer hohen Mischgeschwindigkeit, Sprühen des hergestellten Oberflächenbehandlungsmittels in den Hochgeschwindigkeitsmischer mit einem Sprühbehälter, und gründliches Rühren zum gründlichen Mischen des Oberflächenbehandlungsmittels mit dem Rohmaterialpulver; und
(3) Entnehmen des Rohmaterialpulvers und sofortiges Befördern in einen Ofen, Erwärmen bei 70-105 °C über 4 h, und natürliches Abkühlen vor dem Entnehmen.

3. Verfahren nach Anspruch 1, wobei ein Verfahren der Behandlungen mit laurinsäurehaltigem Lysin die folgenden Schritte umfasst:
(1) Vordispergieren des Rohmaterialpulvers in eine 10%ige bis 15%ige wässrige Lösung;
(2) Einstellen eines pH-Wertes auf etwa 12 mit Natriumhydroxid;
(3) Hinzugeben von 5-10 Gew.-% Pulver von laurinsäurehaltigem Lysin;
(4) Erhöhen einer Lösungstemperatur auf 80 °C und Rühren über 30 min zum Mischen;
(5) Einstellen des pH-Wertes auf 6 mit Schwefelsäure;
(6) Halten der Temperatur, dann Rühren und Umsetzen über 30 min; und
(7) Waschen, Trocknen und Zerkleinern.

4. Verfahren nach Anspruch 1, wobei in dem Schritt (2) das Strahlmahlverfahren das Bearbeiten des vorgemischten Grundierungsmaterials mit Hochdruckluft von 7-8 MPa umfasst.

5. Verfahren nach Anspruch 1, wobei in dem Schritt (4) das vorher hergestellte Farbkonzentrat ein gelbes Konzentrat, ein rotes Konzentrat und ein schwarzes Konzentrat umfasst; wobei das gelbe Konzentrat durch 60 Gew.-% Titandioxid und 40 Gew.-% Eisenoxidgelb gebildet wird; das rote Konzentrat durch 60 Gew.-% Titandioxid und 40 Gew.-% Eisenoxidrot gebildet wird; das schwarze Konzentrat durch 80 Gew.-% Titandioxid und 20 Gew.-% Eisenoxidschwarz gebildet wird.

## Revendications

1. Procédé de préparation d'un matériau de fond de teint pré-mélangé présentant une aberration chromatique minimale, comprenant les étapes suivantes :
(1) processus consistant à soumettre toutes les poudres de matières premières du matériau de fond de teint pré-mélangé à des traitements de surface uniformes de manière à homogénéiser les propriétés de surface des poudres de matières premières, où les poudres de matières premières comprennent des pigments inorganiques et du dioxyde de titane, les traitements de surface comprennent des traitements par un alkylsilicone ou des traitements par la lysine laurique et le pigment inorganique comprend l'oxyde de fer jaune, l'oxyde de fer rouge et l'oxyde de fer noir ;
(2) après les traitements de surface, agitation vigoureuse à haute vitesse des poudres de matières premières pour obtenir le matériau de fond de teint pré-mélangé ; et après mélange au moyen d'un mélangeur à haute vitesse, post-traitement du matériau de fond de teint pré-mélangé par un processus de pulvérisation par jet d'air de manière à disperser uniformément la poudre du matériau de fond de teint pré-mélangé ;
(3) détermination d'une aberration chromatique ΔE_{cmc} du matériau de fond de teint pré-mélangé par comparaison à un échantillon étalon, où un dispositif de mesure sans contact de l'aberration chromatique est utilisé pour mesurer précisément l'aberration chromatique du matériau de fond de teint pré-mélangé ; et
(4) si l'aberration chromatique ΔE_{cmc} n'est pas <0,8, ajustement d'une couleur avec un concentré de couleur pré-préparé, où le concentré de couleur pré-préparé est pré-préparé à l'étape (1) et à l'étape (2).

2. Procédé selon la revendication 1, où un processus de traitement par un alkylsilicone comprend les étapes suivantes :
(1) mélange soigneux de 0,45 kg d'octyltriéthoxysilane et de 1,05 kg d'éthanol à 95 % pour obtenir un agent de traitement de surface immédiatement avant de l'utiliser ;
(2) transfert de 15 kg de la poudre de matière première dans un mélangeur à haute vitesse ; à une vitesse de mélange élevée, pulvérisation de l'agent de traitement de surface préparé dans le mélangeur à haute vitesse au moyen d'un réservoir de pulvérisation et agitation vigoureuse pour mélanger soigneusement l'agent de traitement de surface avec la poudre de matière première ; et
(3) décharge de la poudre de matière première avant transfert immédiat dans un four, chauffage à 70-105 °C pendant 4 h puis refroidissement naturel avant décharge.

3. Procédé selon la revendication 1, où un processus de traitement par la lysine laurique comprend les étapes suivantes :
(1) pré-dispersion de la poudre de matière première pour obtenir une solution aqueuse à 10 %-15 % ;
(2) ajustement d'une valeur de pH à environ 12 avec de l'hydroxyde de sodium ;
(3) addition de 5 - 10 % en poids d'une poudre de lysine laurique ;
(4) augmentation d'une température de la solution à 80 °C et agitation pendant 30 min pour mélanger ;
(5) ajustement de la valeur du pH à 6 avec de l'acide sulfurique ;
(6) maintien de la température puis agitation et mise en réaction pendant 30 min ; et
(7) lavage, séchage et concassage.

4. Procédé selon la revendication 1 où, à l'étape (2), le processus de pulvérisation par jet d'air comprend un traitement du matériau de fond de teint pré-mélangé par de l'air à haute pression (7-8 MPa).

5. Procédé selon la revendication 1 où, à l'étape (4), le concentré de couleur pré-préparé comprend un concentré de jaune, un concentré de rouge et un concentré de noir ; où le concentré de jaune est composé de 60 % en poids de dioxyde de titane et de 40 % en poids d'oxyde de fer jaune ; le concentré de rouge est composé de 60 % en poids de dioxyde de titane et de 40 % en poids d'oxyde de fer rouge ; le concentré de noir est composé de 80 % en poids de dioxyde de titane et de 20 % en poids d'oxyde de fer noir.
